**Europäisches Patentamt**

**European Patent Office**

**Office européen des brevets**

(19)

(11) Veröffentlichungsnummer : **0 502 304 B1**

(12)

# EUROPÄISCHE PATENTSCHRIFT

(45) Veröffentlichungstag der Patentschrift :
**04.08.93 Patentblatt 93/31**

(51) Int. Cl.$^5$ : **A61K 7/09**

(21) Anmeldenummer : **92101205.0**

(22) Anmeldetag : **25.01.92**

(54) **Mittel zur Verformung von menschlichen Haaren enthaltend Alkylpolyglykoside.**

(30) Priorität : **07.03.91 DE 4107313**

(43) Veröffentlichungstag der Anmeldung :
**09.09.92 Patentblatt 92/37**

(45) Bekanntmachung des Hinweises auf die
Patenterteilung :
**04.08.93 Patentblatt 93/31**

(84) Benannte Vertragsstaaten :
**AT BE CH DE DK ES FR GB IT LI NL**

(56) Entgegenhaltungen :
**EP-A- 0 070 074**
**EP-A- 0 362 663**

(73) Patentinhaber : **GOLDWELL
AKTIENGESELLSCHAFT
Zerninstrasse 10-18
W-6100 Darmstadt 13 (DE)**

(72) Erfinder : **Rose, Burkhard
Katharinenstrasse 14
W-6100 Darmstadt 13 (DE)**
Erfinder : **Tennigkeit, Jürgen, Dr.
Felsbergstrasse 51
W-6104 Seeheim Jugenheim (DE)**

EP 0 502 304 B1

**Beschreibung**

Die vorliegende Erfindung betrifft ein Mittel zur dauerhaften Verformung von menschlichen Haaren, d.h., ein Dauerwellmittel, das verbesserte Gebrauchseigenschaften und eine gute Verträglichkeit aufweist.

Bekanntlich erfordert die Dauerwellung zwei Behandlungsschritte:

Die reduktive Spaltung der Cystin-Disulfidbrücken des Haares durch Einwirkung eines Reduktionsmittels und die anschließende Fixierung durch Aufbringung eines Oxidationsmittels, wodurch die Cystin-Disulfidbrücken wieder hergestellt werden.

Das klassische Reduktionsmittel ist dabei die Thioglykolsäure, z.B. als Ammonium- oder Monoethanolaminsalz, die in den letzten Jahren durch Glycerinmonothioglycolat teilweise ersetzt wurde; jedoch werden auch Thiomilchsäure und deren Ester sowie auch anorganische Sulfite eingesetzt.

Die Thioglykolat enthaltenden Zusammensetzungen weisen dabei einen pH-Wert im Bereich zwischen etwa 7,5 und etwa 9,0 auf, wobei die Alkalisierung heute in der Regel, neben Ammoniak, durch Zusatz von Ammonium-(bi)-carbonat erzielt wird.

Die Verformungsmittel auf Basis von Glycerinmonothioglykolat weisen in der Regel einen neutralen pH-Bereich zwischen etwa 6,8 und etwa 7,5 auf.

Die Reduktionsmittel werden in Form von wässrigen Lösungen, Gelen, Emulsionen (Cremes) oder auch als Aerosolschäume eingesetzt und enthalten, neben dem Reduktions- und Alkalisierungsmittel, haarkonditionierende Substanzen, beispielsweise kationische Polymere, gegebenenfalls Verdickungsmittel, sogenannte Carrier, die insbesondere die Penetration des Produktes erhöhen, Komplexbildner, Trübungsmittel, Duftstoffe und, zur Verbesserung des Netz- und Penetrationsvermögens, auch oberflächenaktive Substanzen.

Wesentlicher Bestandteil der Fixier- bzw. Neutralisationsmittel sind Oxidationsmittel. Das am häufigsten benutzte Oxidationsmittel ist Wasserstoffperoxid, dessen Konzentration im Fixierungsmittel üblicherweise zwischen etwa 0,5 und etwa 3 Gew.-% liegt.

Weitere zum Einsatz gelangende Oxidationsmittel sind Alkalibromate, Harnstoffperoxid und Natriumperborat.

Diese Zusammensetzungen werden vorzugsweise in Form wässriger Lösungen oder als Aerosolschäume, seltener als Gele, konfektioniert.

Sie werden auf das Haar, beispielsweise, in Form von Schäumen aufgebracht.

Um das Schäumen zu ermöglichen, enthalten diese Zusammensetzungen, neben verschiedenen anderen Bestandteilen wie Stabilisatoren, Planzenextrakten, haarkonditionierenden Wirkstoffen etc. auch Tenside.

Sowohl bei den in den Reduktionsmittelzusammensetzungen als auch bei den in den Fixiermitteln eingesetzten Tensiden handelt es sich bisher vorzugsweise um die bekannten anionaktiven Produkte, die gegebenenfalls auch in Kombination mit nichtionischen Tensiden zum Einsatz gelangen.

Durch die Verwendung dieser Tenside bzw. Tensidkombinationen wird jedoch keine optimale Ausgewogenheit hinsichtlich des Schaumvermögens und der Netzbarkeit bzw. Penetration einerseits und der Hautverträglichkeit andererseits erreicht.

Die Erfindung geht daher von der Aufgabenstellung aus, ein Mittel zur dauerhaften Verformung von menschlichen Haaren zu schaffen, das sowohl ein optimales Schaum- und Netz- bzw. Penetrationsvermögen als auch eine ausgezeichnete Hautverträglichkeit aufweist.

Diese Aufgabe wird durch die Verwendung von Alkylpolyglykosiden der allgemeinen Formel

$$RO(OR^1)_nZ_x,$$

wobei R eine Alkylgruppe mit 8 bis 20 Kohlenstoffatomen, R1 eine Ethylen-oder Propylengruppe, Z eine Saccharid-Gruppe, n 0 bis 25 und x eine Zahl zwischen 1 and 10 bedeuten, als oberflächenaktive Stoffe in Mitteln zur dauerhaften Verformung von menschlichen Haaren gelöst.

Die erfindungsgemäß zum Einsatz gelangenden Alkylpolyglykoside sind bekannt und in der Literatur beschrieben.

Besonders bevorzugt sind Alkylpolyglykoside, deren Alkylrest zwischen 8 und 18 Kohlenstoffatomen aufweist und deren Kondensationsgrad, für den das Symbol x steht, zwischen 1 und 5, insbesondere zwischen 1,2 und 2 liegt.

Geeignete Alkylpolyglykoside sind beispielsweise Cocoylglykosid (1,7), d.h., mit einem Kondensationsgrad von 1,7, $C_{12}$-$C_{14}$ Alkylpolyglykosid-1,8, Laurypolyglykosid-2, Myristypolyglykosid-2,5, Cetylpolyglykosid-2,2, Stearylpolyglykosid-2, sowie, neben dem bereits genannten Cocoylpolyglykosid,auch Polyglykoside verschiedener gemischter Fettalkohole.

Ein besonders bevorzugtes Alkylpolyglykosid ist ein $C_9$-$C_{11}$-Polyglykosid mit einem Kondensationsgrad von 1,35 sowie eines aus einem $C_{12}$-$C_{16}$ Fettalkoholgemisch mit einem Kondensationsgrad von 1,30 bzw. 1,70.

Auch ein $C_9$-$C_{11}$ Alkylpolyglykosid mit einem Kondensationsgrad-von 1,8 hat sich als geeignet im Rahmen

2

der Erfindung erwiesen.

Geeignete Alkylpolyglykoside und deren Herstellung sind beispielsweise in den US-PSen 3 219 656, 3 598 865, 3 721 633, 3 772 269 und 4 393 203 sowie der EP-A 70 074 beschrieben.

In der letztgenannten Druckschrift wird auch ihre Verwendung in Gemischen mit anionischen Tensiden in Waschmitteln, Geschirrspülmitteln, Stückseifen, Schaumbädern, Shampoos und Rasiercremes geoffenbart; jedoch findet sich keinerlei Hinweis auf die erfindungsgemäße Anwendung in Mitteln zur dauerhaften Haarverformung.

Die Alkylpolyglykoside werden erfindungsgemäß entweder in der Reduktionsmittel-Zusammensetzung oder in der Fixiermittel-Komposition oder in beiden eingesetzt.

Der bevorzugte Mengenanteil in den Fixiermittel-Kompositionen liegt zwischen etwa 0,05 und 5,0, insbesondere 1 bis 2,5 Gew.-% der Gesamtzusammensetzung;in den Reduktionsmittel-Kompositionen können etwa 0,1 bis 5,0 Gew.-% Alkylpolyglykoside, vorzugsweise 0,25 bis 2,5 Gew.-%, anwesend sein.

Die Alkylpolyglykoside können im Rahmen der Erfindung in den Haarverformungsmitteln auch in Kombination mit anderen, bekannten Tensiden eingesetzt werden.

Solche Tenside sind insbesondere die bisher bevorzugt in solchen Zusammensetzungen zum Einsatz gelangenden anionaktiven Stoffe wie Alkylethersulfate und -carboxylate bzw. deren Salze, zusätzliche Schaumverstärker wie $C_8$-$C_{18}$-Fettsäurealkanolamide, beispielsweise Kokosölfettsäuremonoethanolamid, und Aminoxide, beispielsweise Lauryldimethylaminoxid.

Vorzugsweise liegt jedoch in solchen Tensidgemischen das Alkylpolyglykosid im Überschuß vor.

Die erfindungsgemäßen Mittel zur Verformung von menschlichen Haaren weisen im übrigen die bekannten Zusammensetzungen auf und werden nach den bekannten Verfahren hergestellt. Zur Vermeidung von Wiederholungen wird hier auf den Stand der Technik verwiesen, wie er beispielsweise in "Ullmann's Encyclopedia of Industrial Chemistry", Vol. A12 (1986), S.588 bis 591,sowie insbesondere der Monographie von K. Schrader, "Grundlagen und Rezepturen der Kosmetika", 2. Aufl. (1989, Hüthig-Verlag), S.823 bis 840, beschrieben ist.

Die dort geoffenbarten Zusammmensetzungen und Einzelbestandteile, auf die ausdrücklich bezug genommen wird, können auch im Rahmen der vorliegenden Erfindung verwendet werden.

Die folgenden Ausführungsbeispiele dienen der näheren Illustration der Erfindung.


Beispiel 1

a) Reduktionslösung

```
Thioglykolsäure, 80%-ig                    9,0  (Gew.-%)
Ammoniumcarbonat                           5,0
Ammoniak                                   1,7
C12-C14-Alkylpolyglycosid (1,8)            2,0
Parfümöl, Trübungsmittel                   q.s.
Wasser                        ad         100,0
```

b) Fixierlösung

```
Wasserstoffperoxid                         2,2  (Gew.-%)
C10-C14-Alkylpolyglykosid (1,7)            2,0
Parfümöl, Stabilisator                     q.s.
Wasser                        ad         100,0
```

Beide Lösungen weisen gutes Netzvermögen auf, schäumen gut und sind hautverträglich.

Beispiel 2

a) Reduktionslösung

```
Thioglykolsäure, 80%ig                          6,3(Gew.-%)
Ammoniumcarbonat                                4,0
Ammoniak                                        1,1
C9-C11-Alkylpolyglycosid (1,4)                  2,3
Cocoamidopropylbetain                           0,2
1,2-Propandiol                                  5,0
Parfümöl                                         q.s.
Wasser                              ad          100,0
```

b) Fixierlösung

```
Wasserstoffperoxid                              2,3(Gew.-%)
Pentaoxyethylstearylammoniumchlorid             1,3
C9-C11-Alkylpolyglycosid (1,4)                  2,3
Citronensäure                                   0,2
Parfümöl, Acetanilid                            q.s.
Wasser                              ad          100,0
```

Beispiel 3

a) Reduktionsmittel

```
Thiomilchsäure                                  12,5(Gew.-%)
Ammoniak, 25%-ig                                9,0
Ammoniumbicarbonat                              5,0
Harnstoff                                       4,0
Quaternäres kationisches Polymeres              0,5
Nichtionischer Fettsäurepolyglykolester         2,3
Cocobetain                                      0,8
Trübungsmittel, Parfümöl, Pflanzenextrakt       q.s.
Wasser                              ad          100,0
```

b) Fixiermittel

```
Wasserstoffperoxid, 50%-ig                      4,8 (Gew.-%)
Citronensäure                                   0,4
Kamillenextrakt                                 0,3
Na2HPO4                                         0,4
C9-C11-Alkylpolyglucosid(1,3)                   2,0
Natriumpolyglykolethersulfat                    1,5
Parfümöl, Stabilisator,                         q.s.
Perlglanzmittel
Wasser                              ad          100,0
Phosphorsäure zur Einstellung auf pH 3.
```

Beispiel 4

a) Reduktionsmittel

(unmittelbar vor Gebrauch durch Vermischung des Thioglykolsäuremonoglycerinesters mit dem Rest hergestellt)

| | |
|---|---|
| Thioglykolsäuremonoglycerinester | 14,1 (Gew.-%) |
| $C_{12}$-$C_{14}$-Fettalkoholpolyglykolether | 0,4 |
| $C_{10}$-$C_{14}$-Fettalkoholpolyglykosid (2,5) | 0,8 |
| 1,2-Propandiol | 4,5 |
| Komplexbildner (EDTA), Parfümöl | |
| Farbstoff | q.s. |
| Wasser ad | 100,0 |
| pH-Wert auf 6,8 eingestellt | |

b) Fixiermittel

| | |
|---|---|
| Wasserstoffperoxid, 30%-ig | 5,0 (Gew.-%) |
| Natriumlaurylethersulfat | 0,4 |
| $C_{14}$-$C_{16}$-Alkylpolyglykosid (1,5) | 0,9 |
| Parfümöl, Stabilisator | q.s. |
| Wasser ad | 100,0 |

Beispiel 5

a) Reduktionsmittel

| | |
|---|---|
| Thioglykolsäure, 80%-ig | 11,5 (Gew.-%) |
| Ammoniak, 25%-ig | 8,5 |
| Ammoniumcarbonat | 2,0 |
| $C_8$-$C_{12}$-Alkylpolyglykosid (2,0) | 1,0 |
| Ricinusöloxethylat (40 EO) | 1,0 |
| Parfümöl | q.s. |
| Wasser ad | 100,0 |

b) Fixiermittel

| | |
|---|---|
| Natriumbromat | 12,5 (Gew.-%) |
| Harnstoff | 5,2 |
| $C_8$-$C_{16}$-Alkylglykosid (2,0) | 1,8 |
| Parfümöl, Farbstoff, Pflanzenextrakt | q.s. |
| Wasser ad | 100,0 |

Beispiel 6

a) Reduktionsmittel

```
Thioglykolsäure, 80%-ig                          9,5  (Gew.-%)
Ammoniak, 25%-ig                                 6,6
Ammoniumcarbonat                                 1,0
Ricinusöloxethylat (40 EO)                       1,5
C12-C16-Alkylpolyglykosid (1,7)                  1,5
Proteinhydrolysat                                1,0
Parfümöl                                         q.s.
Wasser                              ad           100,0
```

b) Fixiermittel

```
Wasserstoffperoxid                               2,5  (Gew.-%)
Phosphorsäure                                    0,3
C12-C16-Alkylpolyglykosid (1,7)                  2,0
Parfümöl, Stabilisator                          q.s.
Wasser                              ad           100,0
```

Beispiel 7

a) Reduktionsmittel

```
Ammoniumthioglykolat                             5,0  (Gew.-%)
Cystein                                          2,5
Ammoniak                                         0,8
Paraffinöl                                       1,8
C16-C18-Fettalkohol                              4,8
Oelylpolyglykolether                             3,0
Oelylpolyglykosid(1,8)                           0,2
Parfümöl                                        q.s.
Wasser                              ad           100,0
```

b) Fixiermittel

```
Wasserstoffperoxid                               2,5  (Gew.-%)
Cetylstearylalkohol                              2,0
Natriumlaurylethersulfat                         0,2
C12-C16-Alkylpolyglykosid (1,8)                  0,5
Stabilisator, Parfümöl                          q.s.
Wasser                              ad           100,0
```

Beispiel 8

1 Teil einer
Zusammensetzung A aus
70 Gew.-% Glycerinmonothioglykolsäureester, 80%-ig,
20 Gew.-% Glycerinmono-2-thiopropionsäureester,
10 Gew.-% Nonylphenolpolyglykolether
wird mit 3 Teilen einer bis zur Anwendung davon getrennt gehaltenen Zusammensetzung B aus
0,3 Gew.-% Eiweißhydrolysat,
1,5 Gew.-% $C_9$-$C_{11}$-Alkylpolyglykosid (1,3)
0,5 Gew.-% Ricinusölpolyglykolfettsäureester

0,4 Gew.-% Parfümöl
94,8 Gew.-% Wasser,
mit Ammoniak auf pH 7 eingestellt,
vermischt, auf das Haar aufgebracht und 10 bis 30 Minuten einwirken gelassen.
Nach dem Spülen mit Wasser wird mit einer Zusammensetzung C fixiert.

Zusammensetzung C:

```
Wasserstoffperoxid                          2,40  (Gew.-%)
Kationisches Polymer                        0,50
Citronensäure                               0,20
Laurylpolyglycosid (1,5)                    2,00
Na₂HPO₄                                     0,20
Phosphorsäure                               0,15
Stabilisator                                q.s.
Wasser                          ad        100,00
```

Es wird ein glänzendes dauergewelltes Haar ohne jede Reizung der Kopfhaut erhalten.

## Patentansprüche

1. Mittel zur dauerhaften Verformung von menschlichen Haaren, dadurch gekennzeichnet, daß es ein Alkylpolyglykosid der allgemeinen Formel
$$RO(R^1O)_nZ_x,$$
wobei R eine Alkylgruppe mit 8 bis 20 Kohlenstoffatomen, $R^1$ eine Ethylen- oder Propylengruppe, Z eine Saccharid-Gruppe, n 0 bis 25 und x eine Zahl zwischen 1 und 10 bedeutet, enthält.

2. Mittel nach Anspruch 1,dadurch gekennzeichnet, daß R eine Alkylgruppe mit 8 bis 18 Kohlenstoffatomen bedeutet.

3. Mittel nach einem der Ansprüche 1 bis 2, dadurch gekennzeichnet, daß x eine Zahl zwischen 1 und 5 bedeutet.

4. Mittel nach Anspruch 3, dadurch gekennzeichnet, daß x zwischen 1,2 und 2 liegt.

5. Verwendung von Alkylpolyglykosiden der allgemeinen Formel $RO(R^1O)_nZ_x$, worin R eine Alkylgruppe mit 8 bis 20 Kohlenstoffatomen, R1 eine Ethylen- oder Propylengruppe, Z eine Saccharid-Gruppe, n 0 bis 25 und x eine Zahl zwischen 1 und 10 bedeutet, in einem Reduktionsmittel zur Verformung von menschlichen Haaren.

6. Verwendung von 0,1 bis 5 Gew.-%, bezogen auf die Gesamtzusammensetzung, eines Alkylpolyglykosids nach Anspruch 5 in einem Reduktionsmittel zur Verformung von menschlichen Haaren.

7. Verwendung eines Alkylpolyglykosids der allgemeinen Formel $RO(R^1O)_nZ_x$, wobei R eine Alkylgruppe mit 8 bis 20 Kohlenstoffatomen, R1 eine Ethylen- oder Propylengruppe, Z eine Saccharid-Gruppe, n 0 bis 25 und x eine Zahl zwischen 1 und 10 bedeutet, in einem Fixiermittel zur Verformung von menschlichen Haaren.

8. Verwendung von 0,05 bis 5,0 Gew.-%, berechnet auf die Gesamtzusammensetzung, eines Alkylpolyglykosids nach Anspruch 7 in einem Fixiermittel zur Verformung von menschlichen Haaren.

## Claims

1. Composition for permanent waving of human hair, characterized in that it contains an alkyl polyglycoside of the general formula
$$RO(R^1O)_nZ_x,$$

wherein R is an alkyl group with 8 to 20 carbon atoms, $R^1$ is an ethylene or propylene group, Z is a saccharide moiety, n is a number from 0 to 25, and x is a number from 1 to 10.

2. Composition according to claim 1, characterized in that R is an alkyl group with 8 to 18 carbon atoms.

3. Composition according to one of the claims 1 to 2, characterized in that x is a number between 1 and 5.

4. Composition according to claim 3, characterized in that x is a number between 1.2 and 2.

5. Use of alkyl polyglycosides of the general formula $RO(R^1O)_nZ_x$, wherein $R^1$ is an alkyl group with 8 to 20 carbon atoms, $R^1$ is an ethylene or propylene group, Z is a saccharide moiety, n is 0 to 25, and x is a number between 1 and 10, in a reducing composition for waving human hair.

6. Use of 0.1 to 5% by weight, calculated to the total composition, of an alkyl polyglycoside according to claim 5 in reducing compositions for waving human hair.

7. Use of alkyl polyglycosides of the above referred general formula as defined in claim 1, in fixing or neutralizing compositions for waving human hair.

8. Use of 0.05 to 5.0% by weight, calculated to the total composition, of an alkyl polyglycoside according to claim 7 in neutralizing compositions for waving human hair.

## Revendications

1. Composition pour la déformation permanente des cheveux humains caractérisée en ce qu'elle contient un alkylpolyglycoside de formule générale
$$RO(R^1O)_nZ_x$$
où R représente un groupe alkyl avec 8 à 20 atomes de carbone, $R_1$ un groupe éthylène ou propylène, Z un groupe saccharidique, n a une valeur de 0 à 25 et x représente un chiffre compris entre 1 à 10.

2. Composition selon la revendication 1, caractérisée en ce que R représente un groupe alkyl avec 8 à 18 atomes de carbone.

3. Composition selon l'une quelconque des revendications 1 à 2, caractérisée en ce que x représente un chiffre compris entre 1 et 5.

4. Composition selon la revendication 3 caractérisée en ce que x se situe entre 1,2 et 2.

5. Utilisation d'alkylpolyglycosides de formule générale $RO(R^1O)_nZ_x$, ou R représente un groupe alkyl avec 8 à 20 atomes de carbone, $R_1$ un groupe éthylène ou propylène, Z un groupe saccharidique, n a une valeur de 0 à 25 et x représente un chiffre compris entre 1 et 10, mélangés à un agent réducteur, pour la déformation des cheveux humains.

6. Utilisation de 0,1 a 5 % en poids calculé à partir de la composition finale, d'un alkylpolyglycoside selon la revendication 5, mélangé à un agent réducteur pour la déformation des cheveux humains.

7. Utilisation d'un alkylpolyglycoside de formule générale $RO(R^1O)_nZ_x$, ou R représente un groupe alkyl avec 8 à 20 atomes de carbones, $R_1$ un groupe éthylène ou propylène, Z un groupe saccharidique, n étant compris entre 0 et 25, x représente un chiffre compris entre 1 et 10, associé à une composition de fixation pour la déformation des cheveux humains.

8. Utilisation de 0,05 à 5,0 % en poids calculé à partir de la composition finale, d'un alkylpolyglycoside selon la revendication 7 associé à une composition de fixation pour la déformation des cheveux humains.